# FASCICULE DE BREVET EUROPEEN

(11) **EP 1 102 594 B1**
(45) Date de publication et mention de la délivrance du brevet: **15.05.2002**
(21) Numéro de dépôt: 99936674.3
(22) Date de dépôt: 03.08.1999
(51) Int. Cl.: A61K 33/14, A61P 39/00

(54) **UTILISATION DE CHLORURE DE SODIUM POUR REDUIRE LA TOXICITE GASTRO-INTESTINALE DES DERIVES DE LA CAMPTOTHECINE**
VERWENDUNG VON NATRIUMCHLORID ZUR VERMINDERUNG DER GASTROINTESTINALEN TOXIZITÄT VON CAMPTOTECINDERIVATEN.
USE OF SODIUM CHLORIDE TO REDUCE THE GASTROINTESTINAL TOXICITY OF CAMPTOTHECIN DERIVATIVES

(30) Priorité: 05.08.1998 FR 9810043; 12.08.1998 US 96318 P
(43) Date de publication de la demande: 30.05.2001
(73) Titulaire: Aventis Pharma S.A., 92160 Antony (FR)
(72) Inventeur: TOUTAIN, Hervé, F-78140 Velizy-Villacoublay (FR); GUFFROY, Magali, F-94700 Maisons-Alfort (FR)
(74) Mandataire: Le Pennec, Magali
(86) Numéro de dépôt international: FR9901916
(87) Numéro de publication internationale: WO0007605

(56) Documents cités:
- WO-A-96/11005
- US-A- 5 225 404
- WAGENER D.J.T. ET AL: "Phase II trial of CPT-11 in patients with advanced pancreatic cancer, an EORTC early clinical trials group study" ANNALS OF ONCOLOGY(ANN. ONCOL.), 6/2 (129-132), XP002104573 Netherlands

## Description

La présente invention concerne l'utilisation de solutions de chlorure de sodium pour la diminution des effets secondaires gastro-intestinaux entraînés par l'administration de dérivés de la camptothécine.

Il est connu que l'administration de dérivés de la camptothécine provoque de nombreux effets secondaires. Notamment au niveau du tractus gastro-intestinal, ils provoquent le plus souvent des vomissements et des diarrhées très graves pouvant conduire à l'interruption du traitement.

Dans le brevet européen EP 137145 ont été décrits des dérivés de camptothécine de formule générale : 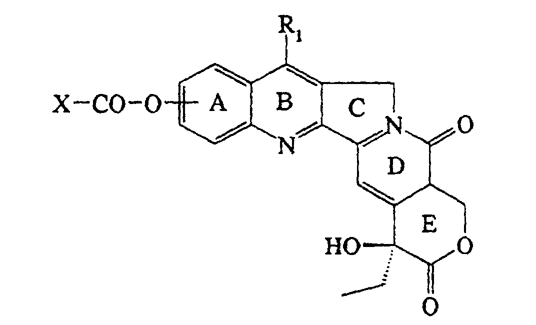 dans laquelle notamment R₁ est hydrogène, halogène ou alcoyle, X est un atome de chlore ou NR₂R₃ pour lequel R₂ et R₃ identiques ou différents peuvent représenter un atome d'hydrogène, un radical alcoyle éventuellement substitué, un carbocycle ou un hétérocycle éventuellement substitués, ou des radicaux alcoyle (éventuellement substitués) formant avec l'atome d'azote auquel ils sont attachés, un hétérocycle contenant éventuellement un autre hétéroatome choisi parmi O, S et/ou NR₄, R₄ étant un atome d'hydrogène ou un radical alcoyle et dans laquelle le groupement X-CO-O-est situé en position 9, 10 ou 11 du cycle A. Ces dérivés de camptothécine sont des agents anticancéreux, inhibiteurs de topoisomérase I, parmi lesquels l'irinotécan, pour lequel X-CO-O- est [4-(1-pipéridino)-1-pipéridino]carbonyloxy, est un principe actif particulièrement efficace sur les tumeurs solides et notamment le cancer colorectal.

Dans la demande de brevet EP 74256 ont également été décrits d'autres dérivés de camptothécine qui sont également mentionnés comme agents anticancéreux, notamment des dérivés de structure analogue à la structure donnée ci-dessus et dans laquelle X-CO-O- est remplacé par un radical -X'R' pour lequel X' est 0 ou S et R' est un atome d'hydrogène, un radical alcoyle ou acyle. D'autres dérivés de camptothécine ont aussi été décrits par exemple dans les brevets ou demandes de brevets EP 56692, EP 88642, EP 296612, EP 321122, EP 325247, EP 540099, EP 737686, WO 9003169, WO 9637496, WO 9638146, WO 9638449, WO 9700876, US 7104894, JP 57 116015, JP 57 116074, JP 59 005188, JP 60 019790, JP 01 249777, JP 01246287, JP 91/12070 ou dans Canc. Res., 38 (1997) Abst. 1526 ou 95 (San Diego - 12-16 avril), Canc. Res., 55(3), 603-609 (1995) ou AFMC Int. Med. Chem. Symp. (1997) Abst. PB-55 (Séoul - 27 juillet-1 août).

Les dérivés de la camptothécine sont habituellement administrés par voie injectable, plus particulièrement par voie intraveineuse sous forme de solution stérile ou d'une émulsion. Les dérivés de la camptothécine peuvent être également administrés par voie orale, sous forme de compositions solides ou liquides.

Les dérivés de camptothécine peuvent aussi être administrés en combinaison avec d'autres agents anticancéreux comme par exemple le cisplatine, l'oxaliplatine, le Tomudex®, le Taxotère®, le 5-fluorouracyl et les inhibiteurs de thymidilate synthase.

Malheureusement, parmi les effets secondaires cliniques liés au traitement par des dérivés de la camptothécine, on note particulièrement l'apparition de diarrhées (de niveau 3 ou 4, de syndrome cholinergique, de nausées ou de vomissements. En particulier chez 38 % des patients des diarrhées sévères sont observées, qui peuvent mettre la vie des patients en danger, par deshydratation et/ou infection associée.

De nombreuses stratégies ont été mises en oeuvre pour lutter contre la toxicité intestinale due à l'administration du principe actif et la diminuer, mais sans succès jusqu'à présent. Il en résulte que les dérivés de camptothécine sont limités à un emploi par des oncologistes très expérimentés et chez seulement certaines catégories de malades pouvant les supporter.

II a maintenant été trouvé, et c'est ce qui fait l'objet de la présente invention, que l'on pouvait obtenir une protection vis à vis des lésions gastro-intestinales induites par un traitement par des dérivés de la camptothécine, grâce à l'administration d'une solution de chlorure de sodium. La protection résulte dans la diminution voire la suppression spécifique des effets secondaires gastro-intestinaux sans que l'exposition systémique au principe actif ou l'activité antitumorale soit diminuée.

La présente invention concerne l'utilisation de chlorure de sodium pour la préparation d'un agent destiné à diminuer ou supprimer les effets secondaires gastro-intestinaux induits par l'administration de dérivés de la camptothécine. Selon l'invention l'agent destiné à diminuer ou supprimer les effets secondaires gastro-intestinaux induits par l'administration de dérivés de la camptothécine est une solution aqueuse de chlorure de sodium.

La protection est obtenue par administration d'une solution de chlorure de sodium simultanément à l'administration du dérivé de camptothécine, ou bien plusieurs jours avant puis simultanément à l'administration du dérivé de camptothécine.

Cet effet surprenant présente une conséquence tout à fait favorable, et notamment permet d'éviter les interruptions de traitement liées aux effets secondaires gastro-intestinaux trop sévères.

Selon l'invention la solution de chlorure de sodium utilisée est une solution aqueuse dont la concentration est comprise entre 4 et 13 g/l. Elle est administrée par voie orale.

De préférence, la solution de chlorure de sodium est utilisée à la concentration de 0,9 g/l.

La solution de chlorure de sodium est préparée selon les méthodes habituelles par dissolution du chlorure de sodium dans de l'eau (eau purifiée, eau stérile par exemple). Elle peut comprendre en outre d'autres agents tels que notamment des agents édulcorants ou aromatisants.

La solution de chlorure de sodium peut être administrée à raison de 5 à 10 ml/kg/administration une ou deux fois par jour, de 5 jours avant le début du traitement à 1 jour après l'arrêt du traitement pour une durée de traitement par le dérivé de camptothécine comprise entre 1 et 5 jours consécutifs. Selon un autre mode d'administration, la solution de chlorure de sodium est administrée à raison de 5 à 10 ml/kg/administration une ou deux fois par jour pendant la durée du traitement, de préférence à raison de 10 ml/kg/administration deux fois par jour, pour une durée de traitement par le dérivé de camptothécine comprise entre 1 et 14 jours consécutifs. De préférence la solution de chlorure de sodium est utilisée selon ce deuxième mode d'administration.

Le dérivé de camptothécine est administré par voie orale, sous forme de compositions solides comme par exemple des gélules dures de gélatine ou de matrice hydrophile semi-solide. Ils peuvent aussi être administrés sous forme de comprimés, de pilules, de gélules, de capsules, de poudres ou de granulés. De préférence les compositions orales peuvent être des comprimés. Dans toutes ces compositions, le produit actif est mélangé notamment à un ou plusieurs diluants ou adjuvants inertes, tels que des sucres, des dérivés de sucres ou des macromolécules hydrophiles, notamment le saccharose, le lactose, le glucose, le maltose, le D-fructose, le sorbitol, des amidons comme l'amidon de blé, l'amidon de maïs ou de riz, la cellulose et ses dérivés comme l'éthyl ou la méthylcellulose, l'hydroxyéthylcellulose, l'hydroxypropylcellulose, la méthylhydroxyméthylcellulose, la méthylhydroxypropylcellulose ou la carboxyméthylcellulose par exemple, ou les gommes comme la gomme arabique, la gomme adragante, la gomme guar, les alginates, les carraghénates ou la dextrine par exemple, ou des protéines, des produits de synthèse comme la polyvinylpyrrolidone, des PEG de haut poids moléculaire, ou encore tels que des produits minéraux comme les silices colloïdales ou les silicates. Ces compositions peuvent comprendre des substances autres, comme par exemple des lubrifiants tels que le stéarate de magnésium ou un enrobage destiné à une libération contrôlée. Ils peuvent également être administrés sous forme de solutions pharmaceutiquement acceptables, de suspensions, d'émulsions, de sirops et d'élixirs contenant des diluants inertes tels que l'eau ou des huiles comme l'huile de paraffine. Ces compositions peuvent également comprendre des substances autres que les diluants, tels que des produits mouillants, édulcorants ou aromatisants comme notamment des sucres ou des polyols.

### Etudes expérimentales :

La présente invention a été mise en évidence lors de différentes études réalisées chez la souris et le chien dans les conditions décrites ci-après.

### Préparation d'une solution injectable :

Une solution pour injection à base de chlorhydrate d'irinotécan trihydraté est préparée, à raison de 20 mg/ml en présence des constituants suivants :
- chlorhydrate d'irinotécan trihydraté 20 mg
- D-sorbitol 45 mg
- acide lactique 0,9 mg
- hydroxyde de sodium qs pH = 3,5
- Eau pour préparations injectables qs 1ml

### 1/ Etudes expérimentales chez la souris

### 1.1/ Etude I

Evaluation de l'effet protecteur d'une solution de chlorure de sodium administrée par voie orale sur la toxicité intestinale du CPT-11 administré par voie intraveineuse.

Des souris mâles CD1 (agées de 5 à 6 semaines) séparés en deux groupes de 10, reçoivent, dans chacun des 2 groupes, une injection intraveineuse (20 ml/kg à raison de 0,5 ml/minute) d'une solution aqueuse de CPT-11 dans du chlorure de sodium à 9 g/l à la dose de 40 mg/kg (environ 120 mg/m2 de surface corporelle) pendant 5 jours consécutifs du jour 6 au jour 10. Cette période de traitement est suivie d'une période de 3 jours sans traitement (jusqu'au jour 13). Dans l'un des groupes, les souris reçoivent en sus une solution aqueuse de chlorure de sodium à 9 g/l à un volume de 20 ml/kg/jour (c'est-à-dire 10 ml/kg/administration, 2 fois par jour à environ 10 heures d'intervalle) par voie orale pendant 10 jours consécutifs (jours 1 à 10) (groupe CPT-11/NaCl). Les animaux du second groupe ne reçoivent pas de chlorure de sodium à 9 g/l par voie orale (groupe CPT-11).

La mortalité et les signes cliniques sont observées tous les jours. Le poids corporel est relevé aux jours 1, 6, 11 et 13. Les souris sont euthanasiées et autopsiées au jour 13. Le tractus gastrointestinal complet est prélevé sur toutes les souris. Celui des souris ayant survécu jusqu'à la fin de la periode d'observation est soumis à un examen microscopique.

La mortalité liée à l'administration de CPT-11 observée aux jours 6 et 7 est observée chez 7/15 souris dans le groupe CPT-11 et chez seulement 2/10 souris dans le groupe CPT-11/NaCl. Les diminutions d'activité motrice, les tremblements, les convulsions et/ou les difficultés respiratoires liées à l'administration de CPT-11 sont observés de manière occasionnelle après l'administration de CPT-11. Des pertes de poids corporel sont observées dans les groupes CPT-11 et CPT-11/NaCI pendant la durée du traitement avec le CPT-11. La perte de poids corporel est plus importante dans le groupe CPT-11 que dans le groupe CPT-11/NaCl et persiste après la fin du traitement dans le groupe CPT-11.

La nature des lésions microscopiques observées dans l'intestin correspond à celles attendues avec un agent anticancéreux. Les lésions prédominent dans l'intestin grêle pour les deux groupes. Ces lésions globalement modérées à marquées se caractérisent par une perte de cryptes et un raccourcissement des villosités. Les lésions intestinales sont moins sévères dans le groupe CPT-11/NaCl, en particulier, des pertes ce cryptes minimales ne sont observées que de façon occasionnelle.

En conclusion, l'administration orale d'une solution isotonique de chlorure de sodium (9 g/l) à 10 ml/kg/administration 2 fois par jour (20 ml/kg/jour) pendant 5 jours avant, et pendant l'administration intraveineuse de CPT-11 pendant 5 jours prévient la survenue des lésions histopathologiques intestinales induites par le CPT-11.

### 1.2/ Etude II

Evaluation de l'effet protecteur de différent régimes d'administration d'une solution de chlorure de sodium par voie orale sur la toxicité intestinale du CPT- 11

Des souris mâles CD1 (agées de 5 ou 6 semaines) sont divisées en 5 groupes de 10 animaux et reçoivent une injection intraveineuse (20 ml/kg à raison de 0,5 ml/minute) d'une solution aqueuse de CPT-11 dans du chlorure de sodium à 9 g/l à la dose de 40 mg/kg (environ 120 mg/m2 de surface corporelle) pendant 5 jours consécutifs du jour 6 au jour 10. Une solution de chlorure de sodium à 9 g/l est administrée deux fois par jour à raison de 10 ml/kg/administration (à environ 10 heures d'intervalle) du jour 1 au jour 10 pour le groupe 1, deux fois par jour à raison de 5 ml/kg/administration du jour 1 au jour 10 pour le groupe 2, une fois par jour à raison de 20 ml/kg/administration du jour 1 au jour 10 pour le groupe 3, deux fois par jour à raison de 10 ml/kg/administration du jour 6 au jour 10 pour le groupe 4. Les animaux du groupe 5 ne reçoivent pas de solution de chlorure de sodium à 9 g/l.

La mortalité et les signes cliniques sont observées tous les jours. Le poids corporel est mesuré aux jours 3, 6, 10 et 13. Les souris sont euthanasiées et autopsiées au jour 13. Le tractus gastrointestinal complet est prélevé sur toutes les souris. Celui des souris ayant survécu jusqu'à la fin de la periode d'observation est soumis à un examen microscopique.

Les signes cliniques observés dans tous les groupes sont similaires à ceux rapportés dans l'etude précédente (étude I). Une diminution de poids corporel est observée dans tous les groupes.

Les lésions microscopiques observées dans l'intestin correspondent à celles attendues avec un agent anticancéreux. Les lésions prédominent dans l'intestin grêle dans tous les groupes. Les lésions intestinales sont moins sévères dans les groupes ayant reçus une solution de chlorure de sodium à 9 g/l par voie orale que dans le groupe traité par le CPT-11 seul. Par contre, l'incidence et la sévérité des lésions intestinales sont identiques dans les groupes 1 à 4 ayant reçus une solution de chlorure de sodium à 9 g/l par voie orale avec un regime d'adminstration différent.

En conclusion, l'administration orale d'une solution isotonique de chlorure de sodium (9 g/l) une ou deux fois par jour à différents volumes d'administration (5 to 20 ml/kg/administration) avant et pendant ou uniquement pendant l'administration intraveineuse de CPT-11 pendant 5 jours prévient de façon comparable la survenue des lésions histopathologiques intestinales induites par le CPT-11.

### 1.3/ Etude III

Evaluation de l'effet d'une solution isotonique de chlorure de sodium administrée par voie orale, sur la toxicité intestinale et systémique du CPT-11 et sur la toxicocinétique du CPT-11 et de son principal métabolite, le SN-38.

Des souris mâles CD1 (agées de 5 à 6 semaines) sont séparés en trois groupes de 10. Les animaux de 2 groupes reçoivent une injection intraveineuse (20 ml/kg à raison de 0,5 ml/minute) d'une solution aqueuse de CPT-11 dans du chlorure de sodium à 9 g/l à la dose de 40 mg/kg (environ 120 mg/m² de surface corporelle) pendant 5 jours consécutifs du jour 1 au jour 5. Cette période de traitement est suivie d'une période de 3 jours sans traitement (jusqu'au jour 8). Dans l'un de ces deux groupes, les souris reçoivent en plus une solution aqueuse de chlorure de sodium à 9 g/l à un volume de 20 ml/kg/jour (c'est à dire 10 ml/kg/administration, 2 fois par jour à environ 10 heures d'intervalle) par voie orale pendant 5 jours consécutifs (jours 1 à 5) (groupe CPT-11/NaCI). Les animaux du second groupe ne reçoivent pas de chlorure de sodium à 9 g/l par voie orale (groupe CPT-11). Dans le troisième groupe n'ayant pas reçu de CPT-11, les souris reçoivent une solution aqueuse de chlorure de sodium à 9 g/l à un volume de 20 ml/kg/jour (c'est à dire 10 ml/kg/administration, 2 fois par jour à environ 10 heures d'intervalle) par voie orale pendant 5 jours consécutifs (jours 1 à 5) (groupe témoin). 36 animaux supplémentaires dans les groupes CPT-11 et CPT-11/NaCl sont utilisés pour la détermination des concentrations plasmatiques de CPT-11 et de son principal métabolite, le SN-38.

La mortalité et les signes cliniques sont observées tous les jours. Le poids corporel est mesuré aux jours 1, 3, 6 et 8. Les prélèvements de plasma pour l'analyse toxicocinétique sont réalisés aux jours 1 et 5. Les souris sont euthanasiées et autopsiées au jour 8. Les poids relatifs et absolus du thymus, de la rate et des testicules sont mesurés et le tractus gastrointestinal complet, la moelle osseuse sternale, le thymus, la rate, les testicules et les épididymes sont prélevés sur toutes les souris et soumis à un examen microscopique.

Les concentrations plasmatiques maximales (Cmax) et les aires sous la courbe (AUC) mesurées pour le CPT-11 et le SN-38, son principal métabolite, sont identiques pour les animaux des groupes CPT-11 et CPT-11/NaCI aux jours 1 et 5.

La mortalité liée à l'administration de CPT-11 observée aux jour 8 est observée chez 1/10 animal dans le groupe CPT-11/NaCI. Les diminutions d'activité motrice et les difficultés respiratoires liées à l'administration de CPT-11 sont observés de manière occasionnelle après l'administration de CPT-11 dans les groupes CPT-11 et CPT-11/NaCl avec une sévérité supérieure dans le groupe CPT-11/NaCl. Des pertes de poids corporel identiques sont observées dans les groupes CPT-11 et CPT-11/NaCl pendant la durée du traitement avec le CPT-11 et persistent après la fin du traitement.

Les lésions microscopiques observées dans le thymus et l'intestin correspondent à celles attendues avec un agent anticancéreux. Les lésions microscopiques observées dans le thymus des animaux des groupes CPT-11 et CPT-11/NaCl sont similaires et se caractérisent par une déplétion lymphoide associée à une diminution du poids et de la taille de cet organe. Les lésions microscopiques intestinales prédominent dans l'intestin grêle pour les groupes CPT-11 et CPT-11/NaCl et se caractérisent principalement par une perte de cryptes et une atrophie des villosités. Les lésions intestinales sont moins sévères dans le groupe CPT-11/NaCl que dans le groupe CPT-11.

En conclusion, l'administration orale d'une solution isotonique de chlorure de sodium 9 g/l deux fois par jour à 10 ml/kg/administration (20 ml/kg/jour) pendant l'administration intraveineuse de CPT-11 pendant 5 jours ne modifie pas l'exposition systémique au CPT-11 et au SN-38, ne module pas la toxicité thymique du CPT-11 mais réduit sélectivement la sévérité des lésions intestinales induites par le CPT-11.

### 1.4/ Etude IV

Evaluation de l'effet de l'administration orale d'une solution de chlorure de sodium sur l'activité antitumorale du CPT-11 administré par voie intraveineuse.

Des souris femelles C3H/HeN porteuses d'un adénocarcinome mammaire MA16/C implanté par voie sous cutanée au jour 1 sont séparés en deux groupes et reçoivent dans chacun des 2 groupes une injection intraveineuse (20 ml/kg) d'une solution aqueuse de CPT-11 dans du glucose à 5% à la dose de 14,6 - 23,6 - 38,0 ou 61,3 mg/kg pendant 5 jours consécutifs du jour 6 au jour 10. Dans un groupe, les souris reçoivent en plus une solution aqueuse de chlorure de sodium à 9 g/l à un volume de 20 ml/kg/jour (c'est à dire 10 ml/kg/administration, 2 fois par jour à environ 10 heures d'intervalle) par voie orale pendant 10 jours consécutifs (jours 1 à 10) (groupe CPT-11/NaCl). Les animaux du second groupe ne reçoivent pas de chlorure de sodium à 9 g/l par voie orale (groupe CPT-11).

L'activité antitumorale du CPT-11 est évaluée à la dose maximale non toxique. Une dose produisant une perte de poids corporel de plus de 20% ou une incidence de mortalité liée à l'administration du CPT-11 de plus de 20% est considérée comme étant trop toxique. Les paramètres évalués inclus l'inhibition de la croissance tumorale (T/C) exprimée en pourcentage, le retard de croissance tumorale (T-C) et le nombre de cellules tumorales tuées (Log tumor cell kill = T-C/3,32 x temps de doublement tumoral). Une valeur de Log tumor cell kill de 0,7 correspond à une activité minimale alors qu'une valeur supérieure à 2,8 correspond à un niveau d'activité élevée.

Pour les animaux du groupe CPT-11, la dose maximale non toxique de CPT-11 est 23,6 mg/kg/day soit une dose totale cumulée de 118 mg/kg, la perte de poids corporel maximale est de 10% au jour 11 et la valeur de Log cell kill est 1,7. Pour les animaux du groupe CPT-11/NaCI, la dose maximale non toxique de CPT-11 est 38,0 mg/kg/jour soit une dose totale cumulée de 190 mg/kg, la perte de poids corporel maximale est de 14,1% au jour 12 et la valeur de Log cell kill est 2,3.

En conclusion, l'administration orale d'une solution isotonique de chlorure de sodium (9 g/l) à 10 ml/kg/administration 2 fois par jour (20 ml/kg/jour) pendant 5 jours avant, et pendant l'administration intraveineuse de CPT11 pendant 5 jours ne diminue pas l'activité antitumorale du CPT-11.

### 2/ Etude expérimentale chez le chien

Evaluation de l'effet protecteur d'une solution de chlorure de sodium administrée par voie orale sur la toxicité intestinale du CPT-11 par voie intraveineuse.

Six chiens femelle beagle (agées de 10 à 12 mois) sont séparés en deux groupes de 3 et reçoivent, dans chacun des 2 groupes, une injection intraveineuse unique (5 ml/kg à raison de 2 ml/minute) d'une solution aqueuse de CPT-11 dans du chlorure de sodium à 9 g/l à la dose de 20 mg/kg (environ 400 mg/m2 de surface corporelle) au jour 6. Dans un groupe, les animaux reçoivent en plus une solution aqueuse de chlorure de sodium à 9 g/l à un volume de 20 ml/kg/jour (c'est à dire 10 ml/kg/administration, 2 fois par jour à environ 8 heures d'intervalle) par voie orale pendant 7 jours consécutifs (jours 1 à 7) (groupe CPT-11/NaCl) de 5 jours avant à 1 jour après l'administration de CPT11. Les animaux du second groupe ne reçoivent pas de chlorure de sodium à 9 g/l par voie orale (groupe CPT-11). Les animaux sont maintenus sans aucune administration pendant 2 jours après la dernière administration de chlorure de sodium à 9 g/l (jusqu'au jour 9).

La mortalité et les signes cliniques sont observées tous les jours. Le poids corporel est relevé durant la période de prétest et aux jours 5 et 9. Les animaux sont euthanasiées et autopsiées au jour 9. Des échantillons représentatifs des tissus prélevés dans le tractus gastrointestinal sont prélevé sur tous les animaux et soumis à un examen microscopique.

Les effets liés à l'administration du CPT-11 durant la période de traitement comprennent de la salivation, de l'agitation, des vomissements (pendant ou immédiatement après l'administration de CPT-11), des diarrhées et une légère perte de poids. L'observation du gros intestin fait apparaître des rougeurs sur la muqueuse qui sont plus fréquentes dans le groupe CPT-11 que dans le groupe CPT-11/NaCl. Les lésions observées résultent d'une altération primaire du compartiment prolifératif et consistent en une dégénérescence de la muqueuse, modérées à marquées dans le groupe CPT-11 et minimales à légères dans le groupe CPT-11/ NaCl.

En conclusion, l'administration intraveineuse d'une dose unique de CPT-11 à des chiens femelles à la dose de 20 mg/kg produit une toxicité gastro-intestinale compatible avec l'activité antimitotique du produit. L'administration orale d'une solution isotonique de chlorure de sodium (9 g/l) à 10 ml/kg/administration 2 fois par jour (20 ml/kg/jour) de 5 jours avant à 1 jour l'administration de CPT-11 prévient la survenue des lésions histopathologiques induites par le CPT-11.

## Revendications

1. Utilisation de chlorure de sodium pour la préparation d'un agent destiné à diminuer ou supprimer les effets secondaires gastro-intestinaux induits par l'administration de dérivés de la camptothécine, **caractérisée en ce que** la solution de chlorure de sodium est administrée par voie orale.

2. Utilisation selon la revendication 1, **caractérisée en ce que** le chlorure de sodium destiné à diminuer ou supprimer les effets secondaires gastro-intestinaux induits par l'administration de dérivés de la camptothécine est une solution aqueuse de chlorure de sodium.

3. Utilisation selon la revendications 2, **caractérisée en ce que** la solution de chlorure de sodium a une concentration comprise entre 4 et 13 g/l.

4. Utilisation selon la revendication 3, **caractérisée en ce que** la solution de chlorure de sodium a une concentration de 9 g/l.

5. Utilisation selon l'une des revendications 3 et 4, **caractérisée en ce que** la solution de chlorure de sodium est administrée à raison de 5 à 10 ml/kg/administration.

6. Utilisation selon l'une des revendications 1 à 5, **caractérisée en ce que** la solution de chlorure de sodium est administrée soit pendant soit avant et pendant l'administration du dérivé de la camptothécine.

7. Utilisation selon la revendication 1, **caractérisée en ce que** le dérivé de la camptothécine est administré par voie injectable ou orale.

## Claims

1. Use of sodium chloride for preparing an agent intended to reduce or eliminate the gastrointestinal side effects induced by the administration of camptothecin derivatives, **characterized in that** the sodium chloride solution is administered orally.

2. Use according to Claim 1, **characterized in that** the sodium chloride intended to reduce or eliminate the gastrointestinal side effects induced by the administration of camptothecin derivatives is an aqueous sodium chloride solution.

3. Use according to Claim 2, **characterized in that** the sodium chloride solution has a concentration of between 4 and 13 g/l.

4. Use according to Claim 3, **characterized in that** the sodium chloride solution has a concentration of 9 g/l.

5. Use according to either of Claims 3 and 4, **characterized in that** the sodium chloride solution is administered in a proportion of 5 to 10 ml/kg/administration.

6. Use according to one of Claims 1 to 5, **characterized in that** the sodium chloride solution is administered either during, or before and during, the administration of the camptothecin derivative.

7. Use according to Claim 1, **characterized in that** the camptothecin derivative is administered by injection or orally.

## Patentansprüche

1. Verwendung von Natriumchlorid zur Herstellung eines Mittels, das zur Verringerung oder Unterdrückung der durch die Verabreichung von Derivaten des Camptothecins induzierten gastro-intestinalen Nebenwirkungen vorgesehen ist, **dadurch gekennzeichnet, daß** die Lösung von Natriumchlorid auf oralem Weg verabreicht wird.

2. Verwendung nach Anspruch 1, **dadurch gekennzeichnet, daß** das zur Verringerung oder Unterdrückung der durch die Verabreichung von Derivaten des Camptothecins induzierten gastro-intestinalen Nebenwirkungen vorgesehene Natriumchlorid eine wäßrige Lösung von Natriumchlorid ist.

3. Verwendung nach Anspruch 2, **dadurch gekennzeichnet, daß** die Lösung von Natriumchlorid eine Konzentration zwischen 4 g/l und 13 g/l besitzt.

4. Verwendung nach Anspruch 3, **dadurch gekennzeichnet, daß** die Lösung von Natriumchlorid eine Konzentration von 9 g/l besitzt.

5. Verwendung nach einem der Ansprüche 3 und 4, **dadurch gekennzeichnet, daß** die Lösung von Natriumchlorid im Verhältnis von 5 bis 10 ml/kg/Verabreichung verabfolgt wird.

6. Verwendung nach einem der Ansprüche 1 bis 5, **dadurch gekennzeichnet, daß** die Lösung von Natriumchlorid entweder während oder vor und während der Verabreichung des Derivates von Camptothecin verabfolgt wird.

7. Verwendung nach Anspruch 1, **dadurch gekennzeichnet, daß** das Derivat von Camptothecin auf dem Injektionsweg oder dem oralen Weg verabreicht wird.
